# EUROPEAN PATENT SPECIFICATION

(11) **EP 3 316 876 B1**
(45) Date of publication and mention of the grant of the patent: **11.03.2020**
(21) Application number: 16739338.8
(22) Date of filing: 29.06.2016
(51) Int. Cl.: A61K 31/047, A61K 31/198, A61K 31/695, A61P 25/28

(54) **ARGININE SILICATE INOSITOL FOR IMPROVING COGNITIVE FUNCTION**
ARGININ-SILICAT-INOSITOL ZUR VERBESSERUNG DER KOGNITIVEN FUNKTION
ARGININE-SILICATE-INOSITOL POUR AMÉLIORATION DE LA FONCTION COGNITIVE

(30) Priority: 30.06.2015 US 201562187120 P
(43) Date of publication of application: 09.05.2018
(73) Proprietor: Nutrition 21, LLC, Purchase, NY 10577 (US)
(72) Inventor: KOMOROWSKI, James, R., Trumbull, CT 06611 (US)
(74) Representative: Hamer, Christopher K.
(86) International application number: PCT/US2016/040128
(87) International publication number: WO 2017/004226

(56) References cited:
- WO-A1-2012/173808
- WO-A2-2004/017913
- US-A- 5 707 970
- CHERIAN LEELA ET AL: "L-arginine and free radical scavengers increase cerebral blood flow and brain tissue nitric oxide concentrations after controlled cortical impact injury in rats", JOURNAL OF NEUROTRAUMA, M.A. LIEBERT, NEW YORK, NY, US, vol. 20, no. 1, 1 January 2003 (2003-01-01), pages 77-85, XP009106134, ISSN: 0897-7151, DOI: 10.1089/08977150360517209

## Description

### CROSS-REFERENCE TO RELATED APPLICATIONS

This application claims priority to U.S. Provisional Patent Application No. 62/187,120, entitled "ARGININE SILICATE INOSITOL FOR IMPROVING COGNITIVE FUNCTION," filed June 30, 2015.

### BACKGROUND

### Field

The present disclosure relates to new uses of an arginine silicate complex. Such complexes are commercially available and sold as, for example, Nitrosigine®. Nitrosigine® has been clinically shown to boost nitric oxide ("NO") levels. Nitric oxide is a key factor in promoting the relaxation of smooth muscle in blood vessels, increasing blood flow to working muscles. In general, the complex is produced by combining arginine, a silicate salt and inositol. Although the compositions described herein generally contain arginine, silicate and inositol, it may be referred to throughout the specification as "inositol-stabilized arginine silicate," "arginine silicate," "arginine silicate inositol," "ASI," "Nitrosigine®," or "complex."

Disclosed herein is the surprising result that ASI can improve a subject's cognitive test results. Accordingly, ASI may be used to improve cognitive functioning in healthy patients. Improved cognitive functioning may be particularly desirable when a subject is fatigued. Fatigue may result from, for example, a lack of sleep and/or after periods of increased mental and/or physical activity. ASI may be administered to patients and may enhance mental focus and/or mental clarity in patients and especially so in fatigued patients. Thus, ASI may be particularly helpful for use in improving the performance of people required to perform complex tasks when rested and/or fatigued.

Such cognitive tests are also generally sensitive to detecting cognitive impairment associated with dementia, for example, Alzheimer's disease. Thus, the present disclosure is also related to the use of ASI for the treatment and/or prevention of cognitive disorders and age-related diseases, disorders, and conditions.

Cognitive disorders may include, for example, stroke, anoxic brain injury, traumatic brain injury, dementia, Alzheimer's disease, Parkinson's disease, dementia, and mild cognitive impairment ("MCI"). MCI may cause cognitive changes that are serious enough to be noticed by those experiencing them or to others, but the changes are not severe enough to consistently interfere with daily life and/or independent function. While age-related diseases, disorders, and conditions, are typically observed in individuals at least 65 years old, early-onset of symptoms may occur many years earlier, even in an individual in their 30's or 40's.

### SUMMARY

Some embodiments include the use of ASI for improving cognition. For example, in some implementations, methods include administering an amount of ASI effective to improve cognition in humans. Some embodiments include the use of ASI for improving mental focus and/or attention span. For example, in some implementations, methods include administering an amount of ASI effective to improve mental focus and/or attention span in humans.

Some embodiments include the use of ASI for improving the performance of complex tasks performed by humans. For example, in some implementations, methods include administering an amount of ASI effective to improve performance of one or more complex tasks. Such improvement may be in the form of decreased time to complete the task in comparison to a baseline. In some aspects, improvement is in the form of a reduced time to learn a complex task in comparison to a baseline. In some aspects improvement is in the form of a reduced number of errors in performance of the complex task in comparison to a baseline. Some implementations involve administration of a composition comprising an effective amount of ASI. The compositions may be formulated for oral delivery. The compositions may be self-administered.

In some aspects, use of ASI for improving the performance of complex tasks performed by humans is more pronounced when the human is fatigued. Thus, ASI may be administered in an amount effective to increase focus, attention span, or cognitive function in fatigued humans. In some aspects, ASI may be used to increase perceived energy levels.

Some embodiments provide for the use of a composition for improving cognitive function, wherein the composition comprises a therapeutically effective amount of an arginine silicate inositol complex. Some embodiments provide for the use of a composition for preparing a medicament for improving cognitive function, wherein the composition comprises a therapeutically effective amount of an arginine silicate inositol complex. Some embodiments provide a composition for use in improving cognitive function, wherein the composition comprises a therapeutically effective amount of an arginine silicate inositol complex.

Some embodiments include the use of ASI for treating and/or preventing cognitive disorders. For example, in some aspects, methods of improving cognitive function include identifying a cognitive disorder in a subject and administering a therapeutically effective amount of an arginine silicate inositol complex. In some embodiments, the identifying includes administering a test that is sensitive to detecting cognitive impairment and/or cognitive diseases or disorders.

In some aspects, the test is sensitive to detecting a disease or disorder selected from the group consisting of one or more of stroke, anoxic brain injury, traumatic brain injury, dementia, Alzheimer's disease, Parkinson's disease, MCI, and age-related memory loss. In some aspects the test is sensitive to attention deficit hyperactivity disorder ("ADHD") or other learning disability.

Some embodiments provide methods for improving cognitive function, comprising administering a first cognitive evaluation to a subject; determining the presence of a cognitive disorder in said subject; administering a therapeutically effective amount of an arginine silicate inositol complex; and administering a second cognitive evaluation on said subject. In some embodiments, said second cognitive evaluation is improved relative to said first cognitive evaluation.

In some embodiments, identifying a cognitive deficit comprises determining whether the subject has a condition (or is likely to have a condition or is predisposed to a condition) selected from stroke, anoxic brain injury, traumatic brain injury, dementia, Alzheimer's disease, Parkinson's disease, MCI, and age-related memory loss, or a combination of the foregoing. In some embodiments, identifying a cognitive deficit comprises determining if a subject has a learning disability or is diagnosed with or displays symptoms of ADHD. For example, the patient may have one or more genetic risk factors (e.g. a mutated gene) and/or a family history of the cognitive condition.

In some embodiments, cognitive evaluations used to identify patient populations are independently selected from the Automatic Trail Making Test (for example, TMT parts A and/or B); Profile of Mood States; Modified Mini-Mental State Examination; Three Word Recall; 7-Minute Screen; AB Cognitive Screen; Addenbrooke's Cognitive Examination (Revised); Abbreviated Mental Test; Brief Alzheimer Screen; Brief Cognitive Scale; Cognitive Abilities Screening Instrument; Cognitive Assessment Screening Test; Cognitive Capacity Screening Examination; Clock Drawing Test; DemTect; Dementia Questionnaire; General Practitioner Assessment of Cognition; Hopkins Verbal Learning Test; Informant Questionnaire on Cognitive Decline in the Elderly; Informant Questionnaire on Cognitive Decline in the Elderly - Short Form; Minnesota Cognitive Acuity Screen; Mini-Cog; Memory Impairment Screen; Mini-Mental State Examination; Mont Montpellier Screen; Neurobehavioral Cognitive Status Examination; Rotterdam Version of the Cambridge Cognitive Examination; Rapid Dementia Screening Test; Short and Sweet Screening Instrument; Symptoms of Dementia Screener; Six Item Screener; Short Memory Questionnaire; Short Orientation Memory Concentration Test; Short Portable Mental Status Questionnaire; Short Test of Mental State; Time and Change; Telephone Interview of Cognitive Status-Modified; Verbal Fluency; Modified WORLD Test, or a combination of the foregoing. In some embodiments, the first and second cognitive evaluations are independently selected from the Automatic Trail Making Test and the Profile of Mood States.

### BRIEF DESCRIPTION OF THE DRAWINGS

**FIG. 1** is a line graph summarizing the results of average patient time to complete trail making test A. ASI treatment patients received 1,500 mg/day. Trail making test A was performed prior to treatment (baseline), on Day 1 of treatment, and on Day 3 of treatment.
**FIG. 2** is a line graph summarizing the results of average patient time to complete trail making test A. ASI treatment patients received 1,500 mg/day. Trail making test A was performed prior to treatment (baseline), on Day 1 of treatment, and on Day 14 of treatment.
**FIG. 3** is a line graph summarizing the results of average patient time to complete trail making test B. ASI treatment patients received 1,500 mg/day. Trail making test B was performed prior to treatment (baseline), on Day 1 of treatment, and on Day 3 of treatment.
**FIG. 4** is a line graph summarizing the results of average patient time to complete trail making test B. ASI treatment patients received 1,500 mg/day. Trail making test B was performed prior to treatment (baseline), on Day 1 of treatment, and on Day 14 of treatment.

While the present invention has been described in some detail for purposes of clarity and understanding, one skilled in the art will appreciate that various changes in form and detail can be made without departing from the true scope of the invention.

### DETAILED DESCRIPTION

The present invention is defined in and by the appended claims. The terminology used in the description presented herein is not intended to be interpreted in any limited or restrictive manner, simply because it is being utilized in conjunction with a detailed description of certain specific embodiments described herein. Furthermore, embodiments described herein can include several novel features, no single one of which is solely responsible for its desirable attributes or which is essential to practicing the embodiments described herein.

As used herein, "identifying," refers to detecting or selecting a subject from a population of potential subjects, for example, to establish that a particular subject possesses certain properties or characteristics. "Identifying" may include, for example, self-identification, self-diagnosis, and diagnosis by a medical professional.

As used herein, "treat," "treatment," or "treating," refers to administering or providing a composition for prophylactic and/or therapeutic purposes.

As used herein, the terms "prophylactic treatment," "prevent," or "preventing," refers to treating a subject who does not yet exhibit symptoms of a disease or condition, but who is susceptible to, or otherwise at risk of, a particular disease or condition, whereby the treatment reduces the likelihood that the patient will develop the disease or condition. A "disorder" is any condition that would benefit from treatment with the compositions described herein.

As used in the claims below and throughout this disclosure, the phrase "consisting essentially of" is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that other elements are optional and can or cannot be present depending upon whether or not they affect the activity or action of the listed elements. For example, the use of a composition "consisting essentially of ASI" for the treatment of a particular disease or disorder would exclude other ingredients that were known to be active in combating the particular disease or disorder.

As used herein, a composition that "substantially" comprises a complex means that the composition contains more than about 80% by weight, more preferably more than about 90% by weight, even more preferably more than about 95% by weight, and most preferably more than about 98% by weight of the complex.

The term "about," unless otherwise stated explicitly herein, means ± 20%. For instance about 100 means 80 to 120, about 5 means 4 to 6, about 0.3 means 0.24 to 0.36, and about 60% means 48% to 72% (not 40% to 80%).

The term "pharmaceutical formulation" refers to preparations which are in such a form as to permit the biological activity of the active ingredients to be effective, and, therefore may be administered to a subject for therapeutic use.

Throughout the specification there are references, for example, to identifying a subject in need of administration of a ASI or in need of treatment for age-related diseases, disorders, and conditions or having a cognitive disorder. The term identification is not intended to be limiting and includes in each instance a belief by the subject that the composition will benefit the subject, self-identification, and identification by third party using various techniques, and is not restricted to a particular age group. The identification may include, but is not limited to, the association or identification with one or more conditions selected from the group consisting of: self-assessment, assessment by a medical professional such as a medical doctor, psychologist, or nurse, assessment by an online testing service, assessment by one or more family members. Identification may include physical examination by a medical doctor, psychologist, or nurse. In some aspects, individuals may self-identify. Identifying may also be accomplished by comparison of test results to relative normal ranges that are normalized for age and/or education. A below average result may result in identification of a need for cognitive improvement.

A "therapeutically effective amount" as used herein includes within its meaning a non-toxic but sufficient amount of a compound active ingredient or composition comprising the same for use in the embodiments disclosed herein to provide the desired therapeutic effect. Similarly "an amount effective to" as used herein includes within its meaning a non-toxic but sufficient amount of a compound active ingredient or composition comprising the same to provide the desired effect. The exact amount of the active ingredient disclosed herein required will vary from subject to subject depending on factors such as the species being treated, the age and general condition of the subject, the severity of the condition being treated, the particular agent being administered, the weight of the subject, and the mode of administration and so forth. Thus, it is not possible to specify an exact "effective amount." However, for any given case, an appropriate "effective amount" may be determined by one of ordinary skill in the art using only routine methods. In some aspects, a therapeutically effective amount may include a dosing regimen. For example, a therapeutically effective amount may include about 1,500 mg of ASI orally consumed each day for three consecutive days. In some aspects, a therapeutically effective amount may include about 1,500 mg of ASI orally consumed each day for fourteen consecutive days. Compositions including ASI may include, for example, between 0.5-5 grams of ASI.

In addition, the appropriate dosage of the compositions will depend, for example, on the condition to be treated, the severity and course of the condition, whether the composition is administered for preventive or therapeutic purposes, previous therapy, the patient's clinical history and response to the composition, the type of composition used, and the discretion of the attending physician. The composition is suitably administered to the patient at one time or over a series of treatments and may be administered to the patient at any time from diagnosis onwards. The composition may be administered as the sole treatment or in conjunction with other drugs or therapies useful in treating the condition in question.

By way of example, a "therapeutically effective amount" of the complex disclosed herein can be, for example, 0.1 µg/kg, 0.5 µg/kg, 1 µg/kg, 1.5 µg/kg, 2.0 µg/kg, 2.5 µg/kg, 3.0 µg/kg, 3.5 µg/kg, 4.0 µg/kg, 4.5 µg/kg, 5.0 µg/kg, 10 µg/kg, 15 µg/kg, 20 µg/kg,25 µg/kg, 30 µg/kg, 35 µg/kg, 40 µg/kg, 45 µg/kg, 50 µg/kg, 55 µg/kg, 60 µg/kg, 65 µg/kg, 70 µg/kg, 75 µg/kg, 80 µg/kg, 85 µg/kg, 90 µg/kg, 95 µg/kg, 100 µg/kg, 150 µg/kg, 200 µg/kg, 250 µg/kg, 300 µg/kg, 350 µg/kg, 400 µg/kg, 450 µg/kg, 500 µg/kg, 550 µg/kg, 600 µg/kg, 650 µg/kg, 700 µg/kg, 750 µg/kg, 80 µg/kg 0, 850 µg/kg, 900 µg/kg, 1 mg/kg, 1.5mg.kg, 2.0 mg/kg, 2.5 mg/kg, 3 mg/kg, 4.0mg/kg, 5.0 mg/kg, 6 mg/kg, 7 mg/kg, 8 mg/kg, 9 mg/kg, 10 mg/kg, 15 mg/kg, 20 mg/kg, 25 mg/kg, 30 mg/kg, 35 mg/kg, 40 mg/kg, 45 mg/kg 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 125 mg/kg, 150 mg/kg, 200 mg/kg, 250 mg/kg, 300 mg/kg, 350 mg/kg, 400 mg/kg, 450 mg/kg, 500 mg/kg, 550 mg/kg, 600 mg/kg, 650 mg/kg, 700 mg/kg, 750 mg/kg, 800 mg/kg, 850 mg/kg, 900 mg/kg, 950 mg/kg, 1g/kg, 5 g/kg, 10 g/kg, or more, or any fraction in between of the ASI complex.

Accordingly, in some embodiments, the dose of the complex in compositions disclosed herein can be about 100 µg to about 100g, preferably per day. For example, the amount of the complex can be 100 µg, 125 µg, 150 µg, 175 µg, 200 µg, 225 µg, 250 µg, 275 µg, 300 µg, 325 µg, 350 µg, 375 µg, 400 µg, 425 µg, 450 µg, 475 µg, 500 µg, 525 µg, 575 µg, 600 µg, 625 µg, 650 µg, 675 µg, 700 µg, 725 µg, 750 µg, 775 µg, 800 µg, 825 µg, 850 µg, 875 µg, 900 µg, 925 µg, 950 µg, 975 µg, 1000 µg, 1.25 g, 1.5 g, 1.75 g, 2.0 g, 2.25 g, 2.5 g, 2.75 g, 3.0 g, 3.25 g, 3.5 g, 3.5 g, 3.75 g, 4.0 g, 4.25 g, 4.5 g, 4.75 g, 5.0 g, 5.25 g, 5.5 g, 5.75 g, 6.0 g, 6.25 g, 6.5 g, 6.75 g, 7.0 g, 7.25 g, 7.5 g, 7.75 g, 8.0 g, 8.25 g, 8.5 g, 8.75 g, 9.0 g, 8.25 g, 9.5 g, 9.75g, 10 g, 20g, 30g, 40g, 50g, 60g, 70g, 80g, 90g, 100g, or more, or any range or amount in between any two of the preceding values. The exemplary therapeutically effective amounts listed above, can, in some embodiments be administered in the methods described elsewhere herein on an hourly basis, e.g., every one, two, three, four, five, six, seven, eight, nine, ten, eleven, twelve, thirteen, fourteen, fifteen, sixteen, seventeen, eighteen, nineteen, twenty, twenty-one, twenty-two, twenty-three hours, or any interval in between, or on a daily basis, every two days, every three days, every four days, every five days, every six days, every week, every eight days, every nine days, every ten days, every two weeks, every month, or more or less frequently, as needed to achieve the desired therapeutic effect.

Advantageously, an effective amount may be between about 2 mg and about 5,000 mg per day. More advantageously, the effective amount is between about 500 mg and about 2,000 mg per day. For the average 70 kg man, this may equate to a dosage of between about 3.6 and 14 mg/kg (250-2,500 mg) and between about 7.1 mg/kg and 14 mg/kg (500 mg-1,000 mg), respectively. Effective doses may be extrapolated from dose-response curves derived from in vitro or animal model test systems. Such animal models and systems are well known in the art.

The present application is directed, in part, to the surprising discovery that arginine and silicate and/or inositol and/or complexes thereof improve cognitive function. Described herein are various dosing amounts, schedules, formulations, and delivery systems for arginine and silicate and/or inositol and/or complexes thereof for use in the treatment and/or prevention of cognitive conditions, the improvement of cognitive function, and/or the increasing of cognitive function.

Some embodiments provide methods of improving cognitive function in an individual in need thereof. In some embodiments, improving cognitive function comprises improving mood in an individual in need thereof. In some embodiments, improving cognitive function comprises improving memory in an individual in need thereof. In some embodiments, improving cognitive function comprises improving abstract reasoning in an individual in need thereof. In some embodiments, improving cognitive function comprises improving perceived energy levels in an individual in need thereof. In some embodiments, improving cognitive function comprises improving attention/working memory in an individual in need thereof. In some embodiments, improving cognitive function comprises improving new verbal learning and recall in an individual in need thereof. In some embodiments, improving cognitive function comprises improving expressive language ability in an individual in need thereof. In some embodiments, improving cognitive function comprises improving visual construction in an individual in need thereof. In some embodiments, improving cognitive function comprises improving executive function in an individual in need thereof.

Some embodiments provide methods of identifying an individual in need of a particular treatment. Some embodiments provide methods of determining whether an individual is known to possess a certain characteristic. As used here, "an individual in need" of treatment refers to an individual having an abnormal characteristic. For example, an individual in need of increased cognitive function is an individual that possess a cognitive defect relative to the general population. Likewise, an individual in need of increased memory is an individual having a memory deficit relative to the general population.

In some embodiments, the vigor-activity is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the fatigue-inertia is decreased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the abstract reasoning capability is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%, as measured in standard abstract reasoning tests.

In some embodiments, the perceived energy level is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the attention/working memory is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, new verbal learning and recall is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the expressive language ability is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, visual construction is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the executive function is increased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, improving cognition comprises improving the speed (i.e., decreasing the time) for a Trail Making Test. In some embodiments, improving cognition comprises decreasing the number of errors in a Trail Making Test. In some embodiments, improving cognition comprises both increasing the speed and decreasing the number of errors in a Trail Making Test.

In some embodiments, the time to complete the test is decreased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the number of errors made during the test is decreased by at least about 10%, or at least about 15%, or at least about 20%, or at least about 25%, or at least about 30% or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or at least about 99%.

In some embodiments, the cognitive improvement is observed after about one week; after about two weeks; after about three weeks; after about four weeks; after about six weeks; after about eight weeks; after about 3 months; after about 4 months; after about 6 months; after about 9 months; after about 12 months; after about 18 months; or after about 24 months.

Enhanced mental flexibility has been shown to benefit athletes when faced with quick decisions and associated adaptations often required during competition, especially in field sports such as football. In addition, the ability to efficiently allocate attention is an important factor for success in all sports. The ability to multitask may help an athlete save energy through more efficient processing, thus also allowing them to perform better than they had previously. Skilled athletes who adapt to rapid changes in visual information are able to allocate their attention more effectively than less skilled athletes. They are then able to use visual scanning techniques as well as speed and anticipation to make changes in their performance. This enhanced mental flexibility allows the athlete to adjust his or her "game" faster than their peers.

Furthermore, a study of female collegiate lacrosse players demonstrated that TMT B scores were positively related to less lacrosse-shot error or less unsuccessful shots. Marsh, D.W., Richard, L.A., Verre, A.B., Myers, J. (2010), Relationships Among Balance, Visual Search, and Lacrosse-Shot Accuracy, J of Str & Cond Res, 24(6):1507-1514. This suggests that successful sports performance encompasses a complex interaction of physical and cognitive skills and that perhaps even a small improvement can make an impact on performance. Therefore, the improvements in complex processing speed with ASI supplementation reported in this study as measured by TMT B support the use of ASI as a cognitive enhancing nutritional ingredient with specific applications in sports, competitions, and other athletic activities. Improvements may also be seen in executive functioning, as well as complex processing speed.

Some embodiments provide methods of improving the cognition of an individual known to possess one or more cognitive defects. Some embodiments provide methods of improving the cognition of an individual having a brain injury. In some embodiments, the brain injury is a traumatic brain injury. In some embodiments, the brain injury is due to an ischemic event, for example, a stroke. In some embodiments, the brain injury is due to brain anoxia.

In some embodiments, the cognitive defect or disorder is identified by one or more physical diagnostic tests, including, but not limited to, physical examination by a medical doctor, nurse, physician's assistant, psychologist, counselor, or other medical professional. In some embodiments, the physical examination comprises self-examination. For example, symptoms of a stroke or head injury may include monoparesis, hemiparesis, quadriparesis, hemisensory decifits, monocular or binocular vision loss, visual loss, visual field deficits, asomia, aphasia, diplopia, facial droop, dysarthria, ataxia, vertigo, and combinations thereof. An individual may self-assess such symptoms and self-report for treatment. In some embodiments, the physical examination comprises one or more medical imaging evaluations, including, but not limited to x-ray, CT scan, PET scan, MRI, and/or fMRI. Diagnosis and/or identification may also be obtained using established guidelines. For example, the DSM-5 may be used, for example, to identify patients having ADHD.

In some embodiments, the individual in need thereof is identified by one or more diagnostic cognitive/mental tests. Certain non-limiting examples of diagnostic cognitive/mental tests include Modified Mini-Mental State Examination (3MS); Three Word Recall (3WR); 7-Minute Screen (7MS); AB Cognitive Screen (ABCS); Addenbrooke's Cognitive Examination (Revised) (ACER); Abbreviated Mental Test (AMT); Brief Alzheimer Screen (BAS); Brief Cognitive Scale (BCS); Cognitive Abilities Screening Instrument (CASI); Cognitive Assessment Screening Test (CAST); Cognitive Capacity Screening Examination (CCSE); Clock drawing test (CDT); Deterioration Cognitive Observee (DECO); DemTect; Dementia Questionnaire (DQ); General Practitioner Assessment of Cognition (GPCOG); Hopkins Verbal Learning Test (HVLT); Informant Questionnaire on Cognitive Decline in the Elderly (IQCODE); Informant Questionnaire on Cognitive Decline in the Elderly - Short Form (IQCODE-SF); Minnesota Cognitive Acuity Screen (MCAS); Mini-Cog; Memory Impairment Screen (MIS); Mini-Mental State Examination (MMSE); Mont Montpellier Screen (MMS); Neurobehavioral Cognitive Status Examination (NCSE); Rotterdam Version of the Cambridge Cognitive Examination (R-CAMCOG); Rapid Dementia Screening Test (RDST); Short and Sweet Screening Instrument (SASSI); Symptoms of Dementia Screener (SDS); Six Item Screener (SIS); Short Memory Questionnaire (SMQ); Short Orientation Memory Concentration Test (S-OMC); Short Portable Mental Status Questionnaire (SPMSQ); Short Test of Mental States (STMS); Time and Change (T&C); Telephone Interview of Cognitive Status-Modified (TICS-M); Trail making Test (TMT); Verbal Fluency - Categories (VFC); Modified WORLD Test (WORLD).

Cognitive/mental tests are used to evaluate an individual's mental state in order to assess cognitive defects, for example, a brain injury, stroke, traumatic brain injury, brain anoxia, dementia, MCI, Alzheimer's disease, Parkinson's disease, ADHD, and/or age-related memory loss. Such cognitive/mental tests may be used alone, or in combination, and may be used alone, or in combination with physical examination.

In some embodiments, the cognitive defect is identified by one or more physical diagnostic tests. In some embodiments, the cognitive defect is identified by one or more cognitive diagnostic tests. In some embodiments, the cognitive defect is identified by a combination of one or more physical diagnostic tests and one or more cognitive diagnostic tests.

The administration of the one or more of the compositions disclosed herein can be by any of the methods of administration described herein or by delivery methods known by one of skill in the art. The compositions may be administered orally, through parenteral nutrition, e.g., feeding tube or intravenously, and through other known means.

For oral administration, the compositions disclosed herein can be provided as a tablet, aqueous or oil suspension, dispersible powder or granule, emulsion, hard or soft capsule, syrup, elixir, or beverage. Compositions intended for oral use can be prepared according to any method known in the art for the manufacture of pharmaceutically acceptable compositions and such compositions may contain one or more of the following agents: sweeteners, flavoring agents, coloring agents and preservatives. The sweetening and flavoring agents will increase the palatability of the preparation. Tablets containing the complexes in admixture with non-toxic pharmaceutically acceptable excipients suitable for tablet manufacture are acceptable. Pharmaceutically acceptable vehicles such as excipients are compatible with the other ingredients of the formulation (as well as non-injurious to the patient). Such excipients include inert diluents such as calcium carbonate, sodium carbonate, lactose, calcium phosphate or sodium phosphate; granulating and disintegrating agents, such as corn starch or alginic acid; binding agents such as starch, gelatin or acacia; and lubricating agents such as magnesium stearate, stearic acid or talc. Tablets can be uncoated or can be coated by known techniques to delay disintegration and absorption in the gastrointestinal tract and thereby provide a sustained action over a longer period of time. For example, a time delay material such as glyceryl monostearate or glyceryl distearate alone or with a wax can be employed.

Formulations for oral use can also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil. Aqueous suspensions can contain the complex of the invention in admixture with excipients suitable for the manufacture of aqueous suspensions. Such excipients include suspending agents, dispersing or wetting agents, one or more preservatives, one or more coloring agents, one or more flavoring agents and one or more sweetening agents such as sucrose or saccharin.

Oil suspensions can be formulated by suspending the active ingredient in a vegetable oil, such as arachis oil, olive oil, sesame oil or coconut oil, or in a mineral oil such as liquid paraffin. The oil suspension can contain a thickening agent, such as beeswax, hard paraffin or cetyl alcohol. Sweetening agents, such as those set forth above, and flavoring agents can be added to provide a palatable oral preparation. These compositions can be preserved by an added antioxidant such as ascorbic acid. Dispersible powders and granules of the invention suitable for preparation of an aqueous suspension by the addition of water provide the active ingredient in admixture with a dispersing or wetting agent, a suspending agent, and one or more preservatives. Additional excipients, for example sweetening, flavoring and coloring agents, can also be present.

Syrups and elixirs can be formulated with sweetening agents, such as glycerol, sorbitol or sucrose. Such formulations can also contain a demulcent, a preservative, a flavoring or a coloring agent.

The complex for parenteral administration can be in the form of a sterile injectable preparation, such as a sterile injectable aqueous or oleaginous suspension. This suspension can be formulated according to methods well known in the art using suitable dispersing or wetting agents and suspending agents. The sterile injectable preparation can also be a sterile injectable solution or suspension in a non-toxic parenterally-acceptable diluent or solvent, such as a solution in 1,3-butanediol. Suitable diluents include, for example, water, Ringer's solution and isotonic sodium chloride solution. In addition, sterile fixed oils can be employed conventionally as a solvent or suspending medium. For this purpose, any bland fixed oil can be employed including synthetic mono or diglycerides. In addition, fatty acids such as oleic acid can likewise be used in the preparation of injectable preparations.

The compositions can also be in the form of oil-in-water emulsions. The oily phase can be a vegetable oil, such as olive oil or arachis oil, a mineral oil such as liquid paraffin, or a mixture thereof. Suitable emulsifying agents include naturally-occurring gums such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsions can also contain sweetening and flavoring agents.

It will be appreciated that the amount of the complex may be combined with a carrier material to produce a single dosage form. Such forms will vary depending upon the host treated and the particular mode of administration.

When administered to a mammal, e.g., to an animal for veterinary use or for improvement of livestock, or to a human for therapeutic use, the compositions disclosed herein are administered in isolated form or as the isolated form in a therapeutic composition. As used herein, "isolated" means that the compositions disclosed herein are separated from other components of either (a) a natural source, such as a plant or cell or food, preferably bacterial culture, or (b) a synthetic organic chemical reaction mixture. Preferably, via conventional techniques, the compositions disclosed herein are purified. As used herein, "purified" means that when isolated, the isolate contains at least 95%, preferably at least 98% of the composition.

In some aspects, arginine silicate may be added to food that is designed for animals. For example, the formulation may be added to and/or comprise a pet treat or biscuit, for example, a dog biscuit or a cat treat

Aqueous suspensions may contain the complexes disclosed herein in admixture with excipients suitable for the manufacture of aqueous suspensions. Such excipients include suspending agents, dispersing or wetting agents, one or more preservatives, one or more coloring agents, one or more flavoring agents and one or more sweetening agents such as sucrose or saccharin.

Oil suspensions may be formulated by suspending the active ingredient in a vegetable oil, such as arachis oil, olive oil, sesame oil or coconut oil, or in a mineral oil such as liquid paraffin. The oil suspension may contain a thickening agent, such as beeswax, hard paraffin or cetyl alcohol. Sweetening agents, such as those set forth above, and flavoring agents may be added to provide a palatable oral preparation. These compositions may be preserved by an added antioxidant such as ascorbic acid. Oil suspensions may be formulated by suspending the active ingredient as a dispersible powder or granule in water, in an admixture with a dispersing or wetting agent, a suspending agent and one or more preservatives. Additional excipients, for example sweetening, flavoring and coloring agents, may also be present. Syrups and elixirs may be formulated with sweetening agents such as glycerol, sorbitol or sucrose. Such formulations may also include a demulcent, a preservative, a flavoring or a coloring agent.

The compositions for parenteral administration may be in the form of a sterile injectable preparation, such as a sterile injectable aqueous or oleaginous suspension. This suspension may be formulated according to methods well known in the art using suitable dispersing or wetting agents and suspending agents. The sterile injectable preparation may also be a sterile injectable solution or suspension in a non-toxic parenterally-acceptable diluent or solvent, such as a solution in 1,3-butanediol. Suitable diluents include, for example, water, Ringer's solution and isotonic sodium chloride solution. In addition, sterile fixed oils may be employed conventionally as a solvent or suspending medium. For this purpose, any bland fixed oil may be employed including synthetic mono- or diglycerides. In addition, fatty acids such as oleic acid may likewise be used in injectable preparations.

The disclosed complexes can also be administered by inhalation. In this administration route, an arginine silicate inositol complex can be dissolved in water or some other pharmaceutically acceptable carrier liquid for inhalation, or provided as a dry powder, and then introduced into a gas or powder that is then inhaled by the patient in an appropriate volume so as to provide that patient with a measured amount of an arginine silicate inositol complex.

Controlled release vehicles are well known to those of skill in the pharmaceutical sciences. The technology and products in this art are variably referred to as controlled release, sustained release, prolonged action, depot, repository, delayed action, retarded release and timed release; the words "controlled release" as used herein is intended to incorporate each of the foregoing technologies.

Numerous controlled release vehicles are known, including biodegradable or bioerodable polymers such as polylactic acid, polyglycolic acid, and regenerated collagen. Known controlled release drug delivery devices include creams, lotions, tablets, capsules, gels, microspheres, liposomes, ocular inserts, minipumps, and other infusion devices such as pumps and syringes. Implantable or injectable polymer matrices, and transdermal formulations, from which active ingredients are slowly released, are also well known and can be used in the disclosed methods.

Controlled release preparations can be achieved by the use of polymers to form complexes with or absorb the arginine silicate inositol complex. The controlled delivery can be exercised by selecting appropriate macromolecules such as polyesters, polyamino acids, polyvinylpyrrolidone, ethylenevinyl acetate, methylcellulose, carboxymethylcellulose, and protamine sulfate, and the concentration of these macromolecule as well as the methods of incorporation are selected in order to control release of active complex.

Controlled release of active complexes can be taken to mean any of the extended release dosage forms. The following terms may be considered to be substantially equivalent to controlled release, for the purposes of the present disclosure: continuous release, controlled release, delayed release, depot, gradual release, long term release, programmed release, prolonged release, programmed release, proportionate release, protracted release, repository, retard, slow release, spaced release, sustained release, time coat, time release, delayed action, extended action, layered time action, long acting, prolonged action, sustained action medications and extended release, release in terms of pH level in the gut and intestine, breakdown of the molecule and based on the absorption and bioavailability.

Hydrogels, wherein an arginine silicate inositol complex is dissolved in an aqueous constituent to gradually release over time, can be prepared by copolymerization of hydrophilic mono-olefinic monomers such as ethylene glycol methacrylate. Matrix devices, wherein an arginine silicate inositol complex is dispersed in a matrix of carrier material, can be used. The carrier can be porous, non-porous, solid, semi-solid, permeable or impermeable. Alternatively, a device comprising a central reservoir of an arginine silicate inositol complex surrounded by a rate controlling membrane can be used to control the release of the complex. Rate controlling membranes include ethylene-vinyl acetate copolymer or butylene terephthalate/polytetramethylene ether terephthalate. Use of silicon rubber depots are also contemplated.

Controlled release oral formulations are also well known. In one embodiment, the active complex is incorporated into a soluble or erodible matrix, such as a pill or a lozenge. In another example, the oral formulations can be a liquid used for sublingual administration. These liquid compositions can also be in the form a gel or a paste. Hydrophilic gums, such as hydroxymethylcellulose, are commonly used. A lubricating agent such as magnesium stearate, stearic acid, or calcium stearate can be used to aid in the tableting process. In a preferred embodiment, transdermal patches, steady state reservoirs sandwiched between an impervious backing and a membrane face, and transdermal formulations, can also be used to deliver an arginine silicate inositol complex. Transdermal administration systems are well known in the art. One type of transdermal patch is a polymer matrix in which the active agent is dissolved in a polymer matrix through which the active ingredient diffuses to the skin.

The amount of a complex that will be effective in the treatment of a particular disorder or condition disclosed herein will depend on the nature of the disorder or condition, and can be determined by standard clinical techniques. In addition, in vitro or in vivo assays may optionally be employed to help identify optimal dosage ranges.

The compositions disclosed herein can preferably be formulated with other active ingredients. For example, compositions disclosed herein may be formulated in combination with donepezil. The transitional phrase "consisting essentially of' limits the scope of a claim to the specified materials or steps and those that do not materially affect the basic and novel characteristics of the claimed invention. Thus, for example, a composition consisting essentially of arginine silicate would not include other ingredients that are known to treat and/or prevent cognitive decline (e.g., donepezil).

The compositions disclosed herein may also be formulated with chromium. Similarly, the methods disclosed herein may include administering an amount of chromium. Chromium may be provided as chromium picolinate, chromic tripicolinate, chromium nicotinate, chromic polynicotinate, chromium chloride, chromium histidinate, chromium nicotinate-glycinate, chromium phenylalanine, chromium yeast, or combinations thereof. Similarly, chromium may be provided as chromium and histidine, a chromium histidinate complex, chromium trihistidinate, a chromium polyhistidinate complex, chromium acetate, chromium picolinate, chromium tripicolinate, chromium chloride, chromium nicotinate, or combinations thereof, including pharmaceutically acceptable salts, hydrates, solvates, or mixtures thereof in combination. Compositions may include 100 µg - 10 g of chromium. In some aspects, compositions are formulated to provide 1,000 µg per day.

The complexes may be administered once, twice, or three times per day. In some aspects, the complexes are administered four times a day. For example, the complexes may be administered before, after, or during a meal.

### EXAMPLES

The compositions comprising ASI (Nitrosigine® - 1,500 mg/day) were tested in two double-blind placebo-controlled crossover-design (DBPC-X) studies using the Trail Making Test (TMT) as the cognitive outcomes measure. TMT A involves connecting a sequence of numbers, whereas TMT B involves connecting an alternating sequence of numbers and letters. Faster times in TMT B are associated with enhanced visual search, speed of processing, mental flexibility, and executive functions under pressure.

### Example 1

The purpose of the first study was to determine the effects of a single dose and 14-days-use of Nitrosigine ® as compared to placebo on cognition by Automated Trail Making Tests A and B.

To assess safety variables, changes in blood pressure and heart rate (from pre-dose to approximately 25 minutes post-dose and with 14-days-use from pre-dose Day 1 to post-dose Day 14) were monitored, along with the occurrence of adverse events (during visits 2 and 4 and with 14-days-use).

An open-label, pharmacokinetic / pharmacodynamic / safety clinical trial was done to characterize the safety, pharmacokinetics, and pharmacodynamics of Nitrosigine®. The study supports the fact that arginine-containing products do increase circulating arginine levels (significant increases in plasma arginine were observed with a single dose and 14-days-use of Nitrosigine®). The study also supports the finding that this increase in circulating arginine increases NO levels. A significant increase in baseline salivary nitrite following 14-days-use of Nitrosigine® was observed, which may support some improvement in NO production.

The study also attempted to determine the safety of 14-days use of Nitrosigine® as compared to placebo on blood pressure, heart rate, adverse events, and subjective remarks. To determine the safety of a single dose of Nitrosigine® as compared to placebo blood pressure and heart rates were sampled. Excluding the screening visit, each subject completing the study participated for approximately 35 days, with 14 days from visit two to visit three and 14 days from visit four to visit five (± three days for visits 3 and 5). Visits three and four were separated by at least seven days (so there was a minimum of seven days between products).

Participants were enrolled, with each subject receiving both test products, in a randomly assigned sequence. Planned enrollment were adult males, aged 18 to 35 years, with BMI 19 to < 29.9 kg/m2, non-smoker, having performed weight training at least three times a week for at least the six months prior to starting the trial, and overall in good health and appropriate for exercise as determined by physical examination, medical history, and electrocardiogram (ECG).

Eligibility was determined at the screening visit (with all screening procedures performed after the Informed Consent process). Screening procedures included determining the subjects' height and weight (BMI of 19 to < 32 kg/m² is inclusionary), reviewing the medical history and current medications, checking vital signs including blood pressure (a blood pressure ≥ 140/90 mmHg was exclusionary), and performing an ECG. Blood was collected at the screening visit and a comprehensive metabolic panel and complete blood count with differential was used for assessing study eligibility. Subjects' one repetition maximum (1-RM) was also estimated.

All eligible subjects were scheduled for two tests (one with active product, one with placebo, in random order), with each test consisting of two visits (baseline and follow-up visits, on Day 1 and Day 14 of taking product ± three days), separated by a minimum of seven days. Subjects took each product for 14 days (± three days). Subjects were instructed to not exercise for the 24 hours prior to visits. Subjects would also be instructed to fast after midnight the night prior to visits.

Subjects were provided a dose of study product at all visits. They were also provided study product to be taken at home for the time between the baseline and follow-up visits. Compliance was measured via the stick pack counting method. By documenting the number of calendar days (including the days of the visits) and the number of stick packs that should have been used during these days, compliance with each product could be calculated.

The product being tested included the following ingredients: arginine, silicate, inositol, and potassium (total carbohydrate 0.9245 grams; ASI 1,500 mg), and the following inactive ingredients: Citric Acid, Maltodextrin, Natural Flavor, Sucralose, Acesulfame Potassium, FD&C Red 40.

The placebo included the following ingredients: Citric Acid, Maltodextrin, Natural Flavor, Sucralose, Acesulfame Potassium, FD&C Red 40 (2.82 grams of carbohydrates).

The first dose of the first product was taken at the first baseline visit. The final dose of the first product was taken at the first follow-up visit. The first dose of the second product was taken at the second baseline visit. The final dose of the second product was taken at the second follow-up visit. For the days between the baseline visits and the follow-up visits, subjects consumed one packet (one stick pack), one time per day. With regard to the timing of consumption of the product: on days without exercise, subjects took the product in the morning, without food; on days with exercise, subjects took the product 30 minutes before they worked out. Subjects were provided a shaker bottle to use for product preparation.

Patients were instructed to shake the packet and cut off top; pour entire contents of stick-pack into dry shaker bottle; pour 10 oz (approximately 300mL) of room-temperature water into shaker bottle; tightly close the flip-cap on the bottle; shake bottle vigorously for 20 seconds; check bottle (especially the bottom) for clumps of powder; repeat steps 4 and 5 until no clumps are visible; with powder completely dissolved, the subject shall consume the entire contents of bottle within five minutes; ice may be added to the beverage as per subject preference; swirl bottle between sips; if residue is left in the bottle, fill with additional 2oz of water and have subject drink.

Study product had to be stored at room-temperature out of direct sunlight. Subjects were instructed to not take the study product the morning of the follow-up visits (visits 3 and 5) because the product had to be taken on-site as part of the visit. Subjects were instructed to bring unused stick packs to the follow-up visits (visits 3 and 5).

To minimize the effect of product sequence (carry-over, training, fatigue, seasonality, etc) on safety and efficacy endpoints, the active product (A) and placebo (B) were administered to each subject in one of the two possible sequences: A-B or B-A. Block-4 randomization was used so that the two possible product sequences were shuffled into random order for assignment to the first four subjects (two subjects being assigned to A-B, and two subjects being assigned to B-A), then shuffled again into a different random permutation for assignment to the next four subjects, and so on for the remaining subjects who were enrolled (plus additional spare product).

The main efficacy analysis was conducted on a Per-Protocol (PP) basis, using the PP population. If, in the judgment of the statistician, an excessive number of subjects were excluded from the PP population, an exploratory analysis could have been conducted on an Intent-to-Treat (ITT) basis, using the ITT population. But attrition was well within the expected limit, so no ITT analysis was necessary.

The first DBPC-X study was conducted in male subjects (N=11 per group) who had moderate exercise routines prior to participating in the study. These subjects took ASI daily for 14 days. Each subject was randomized to receive either double-blind ASI or placebo with a seven day washout period in between. Subjects were tested at baseline and at follow up on day 14 in each period. They were instructed to refrain from exercise for 24 hours prior to testing and to fast after midnight the night prior.

Changes in cognition were measured using the Trail Making Test (TMT). The TMT is one of the most widely used instruments in neuropsychological assessment as an indicator of cognitive processing speed and executive functioning. The test consists of two parts (A and B). The dependent variable for each part is represented by the time to completion of the tasks. TMT A involves connecting an ascending sequence of 25 numbers. TMT B involves connecting an alternating sequence of 25 numbers and letters. Faster times in TMT B are associated with enhanced visual search, speed of processing, mental flexibility, and executive functions under performance demands. It has been proposed that TMT Part B is more sensitive to cognitive flexibility.

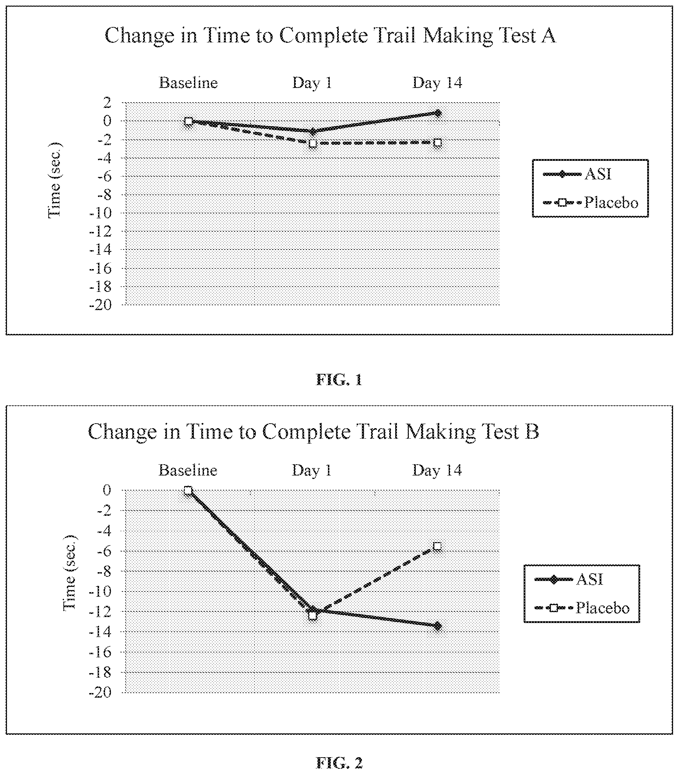

In the first DBPC-X study, changes in TMT A and TMT B were measured pre and post daily doses (approximately 10-15 minutes) in terms of changes from baseline on Day 1 and on Day 14. The results of the first DBPC-X study are shown in **FIGS. 1-2****.** Mean times for TMT A and TMT B can be seen in **Tables 1-2.**

**Table 1. - TMT A Times (see ± SD) Study #1**

| | **Baseline** | **Day 1** | **Day 14 (pre-dose)** |
|---|---|---|---|
| ASI | 16.4 ± 6.7 | 15.8 ± 7.3 | 17.8 ± 8.8 |
| Placebo | 18.3 ± 8.7 | 15.9 ± 3.8 | 16.0 ± 7.1 |

**Table 2 - TMT B Times (sec ± SD) Study #1**

| | **Baseline** | **Day 1** | **Day 14 (pre-dose)** |
|---|---|---|---|
| ASI | 47.3 ± 29.7 | 35.5 ±18.5 | 33.9 ± 13.1 |
| Placebo | 43.6 ± 17.8 | 31.3 ± 10.1 | 38.2 ± 18.8 |

Additional TMT B data is shown in Table 3.

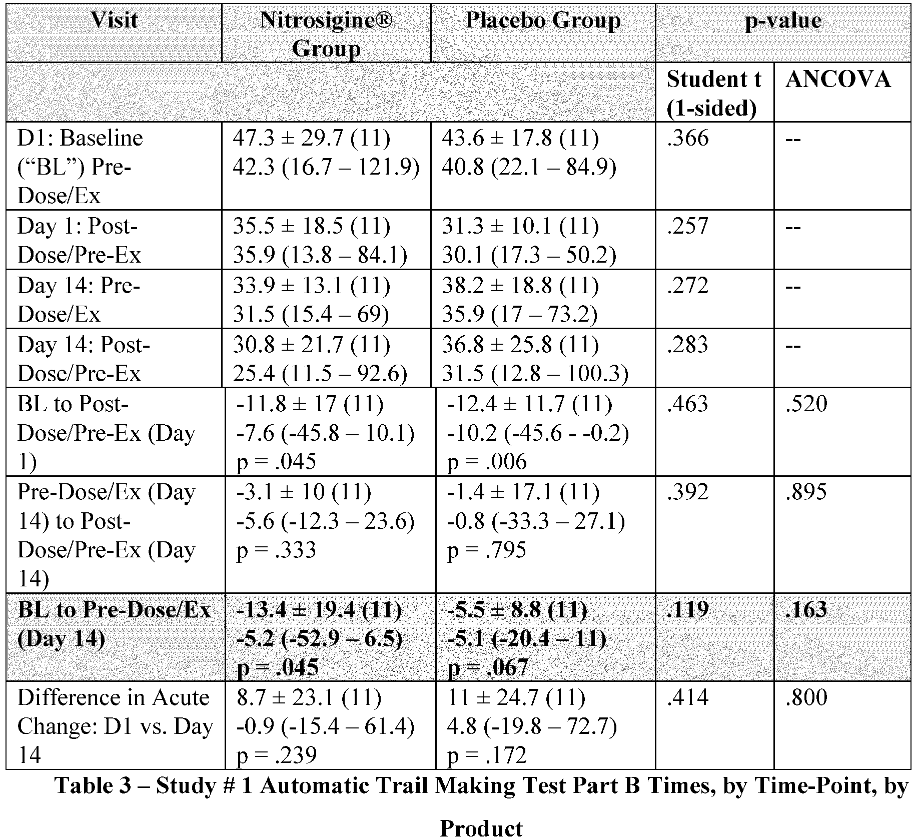

As shown in **Table 3**, TMT B time significantly decreased after 14 days of treatment by 13.4 seconds in the ASI group (p=0.045) from a baseline time of 47.3 seconds (a 28% improvement), compared to a non-significant decrease of 5.5 seconds in the placebo group (p=0.067). While TMT A times did not improve, the significant improvement in TMT B test times preliminarily suggests improved complex processing speed in subjects treated with ASI. Other cognitive domains may be improved as well.

### Example 2

The purpose of this study was to determine the effects of a single dose and four-days-use of Nitrosigine® as compared to placebo on cognition as measured by Automated Trail Making Tests A and B.

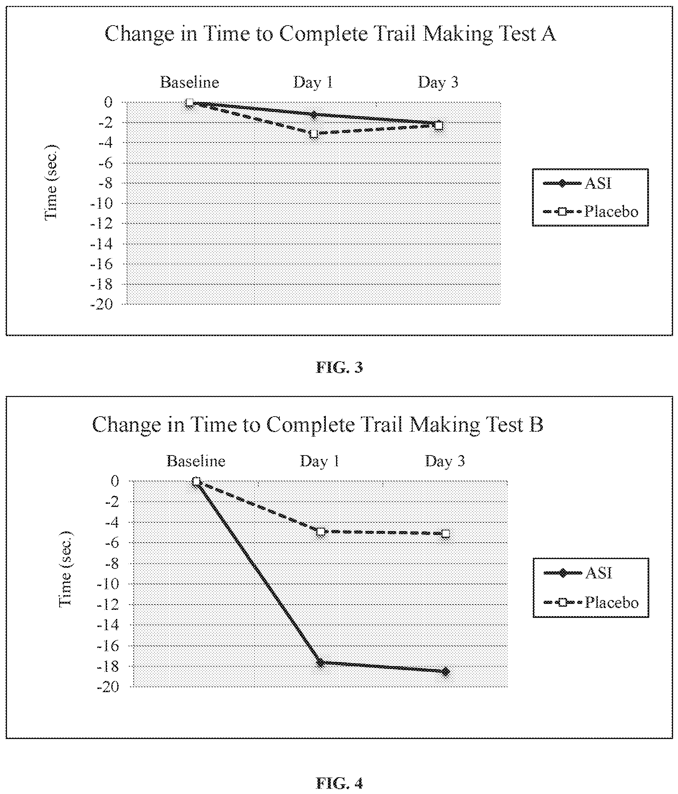

The second DBPC-X study was conducted in male subjects (population described above in Example 1; N=16 per group) who had limited exercise routines prior to participating in the study. These subjects took ASI daily for 3 days. The results of the first DBPC-X study are shown in **FIGS. 3-4****.** Mean times for TMT A and TMT B can be seen in **Tables 4-5.**

**Table 4 - TMT A Times (sec ± SD) Study #2**

| | **Baseline** | **Day 1** | **Day 3 (pre-dose)** |
|---|---|---|---|
| ASI | 20.7 ± 5.1 | 19.5 ± 6.5 | 18.6 ± 6.2 |
| Placebo | 22.8 ± 8.6 | 19.7 ± 7.6 | 20.6 ± 6.5 |

**Table 5 - TMT B Times (sec ± SD) Study #2**

| | Baseline | Day 1 | Day 3 (pre-dose) |
|---|---|---|---|
| ASI | 52.7 ± 21.1 | 40.8 ± 19.4 | 38.0 ± 17.2 |
| Placebo | 59.1 ± 26.3 | 54.8 ± 28.8 | 54.0 ± 44.7 |

Additional TMT B data is shown in **Table 6.**

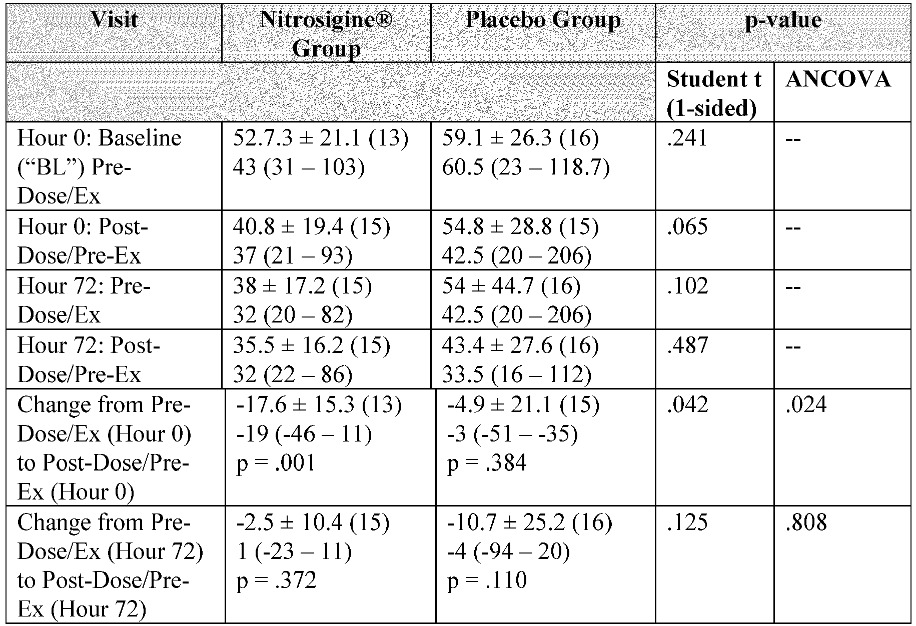

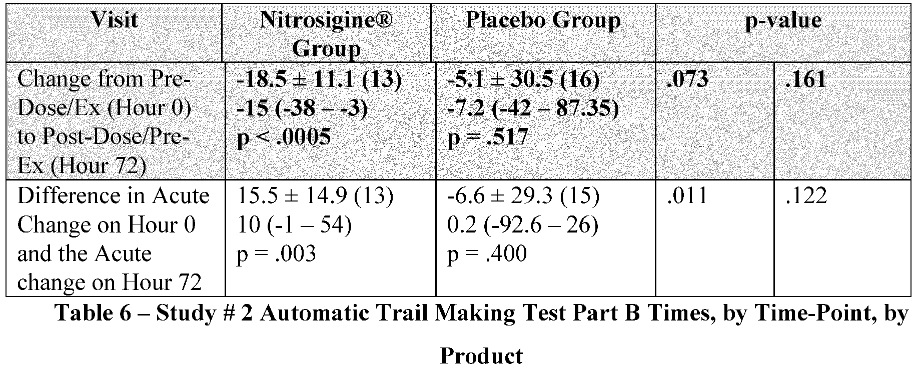

Approximately 10 minutes after taking the first dose, TMT B time decreased by 17.6 seconds in the ASI group (p=0.001) from a baseline time of 52.7 seconds (a 33% improvement), compared to a decrease of 4.9 seconds in the placebo group (p=0.384). The changes in TMT B times after 10 minutes were statistically significant between groups (p=0.024). After 3 days of dosing, TMT B time decreased 18.5 seconds compared to baseline (p<0.0005) a 35% improvement, whereas the placebo group decreased 5.1 seconds (p=0.517). No significant changes were seen in TMT A times in either group. No safety concerns were raised by this study.

These findings show that daily doses of ASI significantly improved TMT B times, with effects seen in as little as 10 minutes after dosing and continued improvement over 14 days. Nitrosigine led to a significant decrease in TMT B time compared to placebo, with an effect size of d=0.80, which shows the strength of the effect to be considerable. Improvement in TMT B test times suggests improved complex processing speed in subjects treated with ASI.

While expected practice effects were seen in the placebo group, the reductions in completion times seen with ASI supplementation were greater than would be expected from simple practice effects alone. Reductions in completion times seen with ASI supplementation had effect sizes of d=0.45, d=0.70 and d=0.80, which shows the strength of the effect to be considerable (from medium to large effect). The data also demonstrates benefits seen with acute supplementation, within 10 minutes of dosing, which may suggest a benefit immediately prior to competition or exercise. Performing TMT B requires executive functioning, speed and working memory. It should be noted that no safety issues were seen with single and repeated use over 14 days.

### Example 3: Randomized, Crossover, Placebo-Controlled Clinical Trial Evaluating Administration of ASI to Individuals with Cognitive Deficits

In a randomized, crossover, placebo-controlled trial, 50 subjects are screened for cognitive deficits based on self-reporting or reporting by friends or family members. The subjects are screened using a battery of four cognitive assessments: the Modified Mini-Mental State Examination (3MS), the Cognitive Abilities Screening Instrument (CASI), the Brief Alzheimer Screen (BAS), and the Short Memory Questionnaire (SMQ). Based on the results of this evaluation, 35 subjects are selected to proceed in the trial.

The arginine silicate inositol complex is prepared as described in the examples above, and is self-administered twice per day in 1 gram doses, for a total of 2 grams of the complex per day in Study Group A. Study Group B also receives the complex twice per day in 1 gram doses, for a total of 2 grams per day in combination with donepezil (a total of 10 mg/day). Study Group C receives the complex twice per day in 1 gram doses, for a total of 2 grams per day in combination with chromium (a total of 500 µg/day). The Control Group receives 2 grams of a placebo complex (inositol alone) per day in 1 gram doses.

Subjects are evaluated using the same panel of cognitive tests at one week, four weeks, eight weeks, twelve weeks, and sixteen weeks.

The Control Group shows no significant improvement at any time point. Study Groups A and B and C exhibit significant cognitive improvement on each cognitive panel starting at the one week time point, with a gradual increase over time, through the end of the study. Individuals in Study Group B who score poorly on the BAS show the greatest improvement. Study Group C performs better than Study Group A.

## Claims

1. Inositol-stabilized arginine silicate for use in treating and/or preventing a cognitive disorder in a subject.

2. The inositol-stabilized arginine silicate for use according to claim 1, wherein the subject is a human.

3. The inositol-stabilized arginine silicate for use according to claim 1 or claim 2, wherein the cognitive disorder is selected from stroke, anoxic brain injury, traumatic brain injury, dementia, Alzheimer's disease, Parkinson's disease, MCI, age-related memory loss and ADHD.

4. The inositol-stabilized arginine silicate for use according of any of claims 1-3, wherein the amount of inositol-stabilized arginine silicate administered is between 0.5 g to 5 g per day, for example 1,500 mg per day.

5. The inositol-stabilized arginine silicate for use according to any of claims 1-3, wherein the inositol-stabilized arginine silicate is administered orally or is self-administered.

6. The inositol-stabilized arginine silicate for use according to any of claims 1-5, wherein an amount of a chromium containing complex is also administered.

7. The inositol-stabilized arginine silicate for use according to any of claims 1-6, wherein the treatment and/or prevention of cognitive disorders further comprises administering a first and a second cognitive evaluation on the subject before and after administration of the inositol-stabilized arginine silicate respectively, preferably wherein said second cognitive evaluation is improved relative to said first cognitive evaluation.

8. A composition comprising inositol-stabilized arginine silicate for use in treating and/or preventing a cognitive disorder in a subject.

9. A non-therapeutic method of improving cognitive function in a subject comprising administering inositol-stabilized arginine silicate to the subject.

10. The non-therapeutic method of claim 9, wherein the subject is a mentally and/or physically fatigued human.

11. The non-therapeutic method of claim 9 or claim 10, wherein the amount of inositol-stabilized arginine silicate administered is between 0.5 g to 5 g per day, for example 1,500 mg per day.

12. The non-therapeutic method of any of claims 9-11, wherein the inositol-stabilized arginine silicate is administered orally and/or is self-administered.

13. The non-therapeutic method of any of claims 9-12, wherein an amount of a chromium containing complex is also administered.

14. Non-therapeutic use of inositol-stabilized arginine silicate for improving cognitive function in a subject.

15. Non-therapeutic use of a composition comprising inositol-stabilized arginine silicate for improving cognitive function in a subject.

## Patentansprüche

1. Inositol-stabilisiertes Arginin-Silikat zur Verwendung bei der Behandlung und/oder Prävention einer kognitiven Störung bei einem Patienten.

2. Inositol-stabilisiertes Arginin-Silikat zur Verwendung nach Anspruch 1, wobei der Patient ein Mensch ist.

3. Inositol-stabilisiertes Arginin-Silikat zur Verwendung nach Anspruch 1 oder Anspruch 2, wobei die kognitive Störung ausgewählt ist aus einem Schlaganfall, einer anoxischen Hirnverletzung, einer traumatischen Hirnverletzung, einer Demenz, Morbus Alzheimer, Morbus Parkinson, einer leichten kognitiven Beeinträchtigung (MCI; *mild cognitive impairment*), einem altersbedingten Gedächtnisverlust und einer Aufmerksamkeitsdefizit-Hyperaktivitätsstörung (ADHS/ADHD; *attention-deficit hyperactivity disorder*).

4. Inositol-stabilisiertes Arginin-Silikat zur Verwendung nach einem der Ansprüche 1 bis 3, wobei die verabreichte Menge von Inositol-stabilisiertem Arginin-Silikat zwischen 0,5 g bis 5 g pro Tag, zum Beispiel 1500 mg pro Tag beträgt.

5. Inositol-stabilisiertes Arginin-Silikat zur Verwendung nach einem der Ansprüche 1 bis 3, wobei das Inositol-stabilisierte Arginin-Silikat oral verabreicht wird oder selbst verabreicht wird.

6. Inositol-stabilisiertes Arginin-Silikat zur Verwendung nach einem der Ansprüche 1 bis 5, wobei auch eine Menge eines Chrom enthaltenden Komplexes verabreicht wird.

7. Inositol-stabilisiertes Arginin-Silikat zur Verwendung nach einem der Ansprüche 1 bis 6, wobei die Behandlung und/oder Prävention von kognitiven Störungen weiter die Vornahme einer ersten und einer zweiten kognitiven Bewertung an dem Patienten vor bzw. nach der Verabreichung des Inositol-stabilisierten Arginin-Silikats umfasst, bevorzugt wobei die genannte zweite kognitive Bewertung relativ zur genannten ersten kognitiven Bewertung besser ausfällt.

8. Zusammensetzung, umfassend Inositol-stabilisiertes Arginin-Silikat zur Verwendung bei der Behandlung und/oder Prävention einer kognitiven Störung bei einem Patienten.

9. Nicht-therapeutisches Verfahren zur Verbesserung der kognitiven Funktion bei einem Patienten, umfassend die Verabreichung von Inositol-stabilisiertem Arginin-Silikat an den Patienten.

10. Nicht-therapeutisches Verfahren nach Anspruch 9, wobei der Patient ein mental und/oder physisch erschöpfter Mensch ist.

11. Nicht-therapeutisches Verfahren nach Anspruch 9 oder Anspruch 10, wobei die verabreichte Menge von Inositol-stabilisiertem Arginin-Silikat zwischen 0,5 g bis 5 g pro Tag, zum Beispiel 1500 mg pro Tag beträgt.

12. Nicht-therapeutisches Verfahren nach einem der Ansprüche 9 bis 11, wobei das Inositol-stabilisierte Arginin-Silikat oral verabreicht wird und/oder selbst verabreicht wird.

13. Nicht-therapeutisches Verfahren nach einem der Ansprüche 9 bis 12, wobei auch eine Menge eines Chrom enthaltenden Komplexes verabreicht wird.

14. Nicht-therapeutische Verwendung von Inositol-stabilisiertem Arginin-Silikat zur Verbesserung der kognitiven Funktion bei einem Patienten.

15. Nicht-therapeutische Verwendung einer Zusammensetzung, umfassend Inositol-stabilisiertes Arginin-Silikat zur Verbesserung der kognitiven Funktion bei einem Patienten.

## Revendications

1. Silicate d'arginine stabilisé à l'inositol destiné à être utilisé dans le traitement et/ou la prévention d'un trouble cognitif chez un sujet.

2. Silicate d'arginine stabilisé à l'inositol destiné à être utilisé selon la revendication 1, dans lequel le sujet est un humain.

3. Silicate d'arginine stabilisé à l'inositol destiné à être utilisé selon la revendication 1 ou la revendication 2, dans lequel le trouble cognitif est sélectionné parmi une attaque, une lésion cérébrale anoxique, une lésion cérébrale traumatique, la démence, la maladie d'Alzheimer, la maladie de Parkinson, une déficience cognitive légère, la perte de mémoire liée à l'âge et le TDAH.

4. Silicate d'arginine stabilisé à l'inositol destiné à être utilisé selon l'une quelconque des revendications 1 à 3, dans lequel la quantité administrée de silicate d'arginine stabilisé à l'inositol est entre 0,5 g à 5 g par jour, par exemple 1 500 mg par jour.

5. Silicate d'arginine stabilisé à l'inositol destiné à être utilisé selon l'une quelconque des revendications 1 à 3, dans lequel le silicate d'arginine stabilisé à l'inositol est administré par voie orale ou est auto-administré.

6. Silicate d'arginine stabilisé à l'inositol destiné à être utilisé selon l'une quelconque des revendications 1 à 5, dans lequel une quantité d'un complexe contenant du chrome est aussi administrée.

7. Silicate d'arginine stabilisé à l'inositol destiné à être utilisé selon l'une quelconque des revendications 1 à 6, dans lequel le traitement et/ou la prévention de troubles cognitifs comprennent en outre l'administration d'une première et d'une deuxième évaluations cognitives sur le sujet avant et après l'administration du silicate d'arginine stabilisé à l'inositol respectivement, préférablement dans lequel ladite deuxième évaluation cognitive est améliorée par rapport à ladite première évaluation cognitive.

8. Composition comprenant du silicate d'arginine stabilisé à l'inositol destinée à être utilisée dans le traitement et/ou la prévention d'un trouble cognitif chez un sujet.

9. Procédé non thérapeutique d'amélioration de la fonction cognitive chez un sujet comprenant l'administration de silicate d'arginine stabilisé à l'inositol au sujet.

10. Procédé non thérapeutique selon la revendication 9, dans lequel le sujet est un humain fatigué mentalement et/ou physiquement.

11. Procédé non thérapeutique selon la revendication 9 ou la revendication 10, dans lequel la quantité administrée de silicate d'arginine stabilisé à l'inositol est entre 0,5 g à 5 g par jour, par exemple 1 500 mg par jour.

12. Procédé non thérapeutique selon l'une quelconque des revendications 9 à 11, dans lequel le silicate d'arginine stabilisé à l'inositol est administré par voie orale et/ou est auto-administré.

13. Procédé non thérapeutique selon l'une quelconque des revendications 9 à 12, dans lequel une quantité d'un complexe comprenant du chrome est aussi administré.

14. Utilisation non thérapeutique de silicate d'arginine stabilisé à l'inositol destinée à améliorer la fonction cognitive chez un patient.

15. Utilisation non thérapeutique d'une composition comprenant du silicate d'arginine stabilisé à l'inositol destinée à améliorer la fonction cognitive chez un patient.
